# EUROPEAN PATENT SPECIFICATION

(11) **EP 3 736 259 B1**
(45) Date of publication and mention of the grant of the patent: **03.08.2022**
(21) Application number: 19305606.6
(22) Date of filing: 10.05.2019
(51) Int. Cl.: C07C 45/69, C07C 45/65, C07C 49/567, C07C 49/813, C08G 65/40

(54) **NEW CYCLOADDUCT PRECURSORS OF DIHALOBENZOPHENONES AND PREPARATIONS THEREOF**
NEUE CYCLOADDUKTVORLÄUFER VON DIHALOGENBENZOPHENONEN UND PRÄPARATE DAVON
NOUVEAUX PRÉCURSEURS CYCLOADDUITS DE DIHALOBENZOPHÉNONES ET LEURS PRÉPARATIONS

(43) Date of publication of application: 11.11.2020
(73) Proprietor: RHODIA OPERATIONS, 69003 Lyon (FR)
(72) Inventor: MULLER, Eric, 69003 Lyon (FR)
(74) Representative: Valentino, Cédric

(56) References cited:
- WO-A1-2007/014692
- US-A- 5 777 172

## Description

### TECHNICAL FIELD

The present invention pertains, as new and useful chemical compounds, to specific di-(halo-cyclohexenyl)ketones, obtainable by cycloaddition between a divinylketone and a 2-halobutadiene (Diels-Alder reaction) and to their use as precursors for the preparation of dihalobenzophenones.

### BACKGROUND ART

Dihalobenzophenones are valuable chemical compounds which are known to the art. They are mainly prepared by oxidation of the methylene group of a dihalodiphenyl methane to provide the corresponding dihalobenzophenone. Various other methods are known, such as those described in US 4943358, WO 2012/001131, US 5777172 and in Dunlop et al., "The preparation of 4-fluoro- and 4,4'-difluorobenzophenone", J. Am. Chem. Soc., 1933, 55(4), pp. 1665-1666. WO 2007/014692 describes a process for preparing 4,4'-difluorobenzophenone, wherein, in a first step, fluorobenzene is reacted with formaldehyde under catalysis by organic sulphonic acids to give difluorodiphenylmethane, the product obtained is isolated, and, in a second step, oxidized with nitric acid to give 4,4'-difluorobenzophenone. A non-exhaustive summary of other available processes for the manufacture of 4,4'-difluorobenzophenone may also be found in US 7687668.

4,4'-difluorobenzophenone (4,4'-DFBP) is the central starting material for preparing poly(aryl ether ketone)s (PAEK) which are a well-known class of engineering polymers used in various fields. These are high-performance polymers with constantly growing annual production volumes, so that the volume of 4,4'-DFBP produced worldwide annually is also included in the growth. The most important PAEK are the poly(ether ketone) (PEK) and poly(ether ether ketone) (PEEK), which feature melting points of above 330°C and high chemicals resistance.

The present invention aims at providing a new and efficient process for the preparation of dihalobenzophenones.

Another objective of the present invention is to provide a process for the preparation of dihalobenzophenones involving renewable starting materials.

### SUMMARY OF THE INVENTION

The present invention therefore relates to a compound of formula (I): 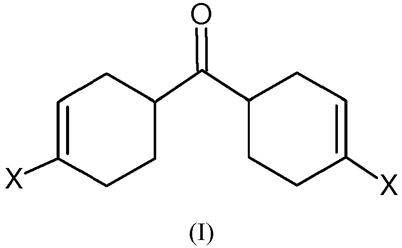 wherein X represents a halogen atom selected from the group consisting of fluorine, chlorine, bromine and iodine.

Preferably, the halogen atom is selected from chlorine and fluorine.

Another object of the invention relates to a process for the preparation of a compound of formula (I) as defined above, comprising reacting divinylketone with a 2-halobutadiene of formula (II): 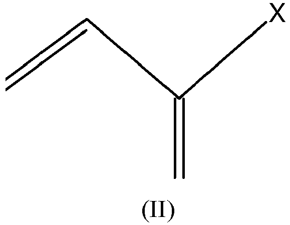 wherein X is as defined above,
and optionally further isolating compound of formula (I).

Preferably, the 2-halobutadiene is 2-chlorobutadiene or 2-fluorobutadiene.

According to an embodiment, the molar ratio of the 2-halobutadiene of formula (II) to divinylketone is of at least 2/1.

The present invention further relates to the use of a compound of formula (I) as defined above, for the preparation of a 4,4'-dihalobenzophenone of formula (III): 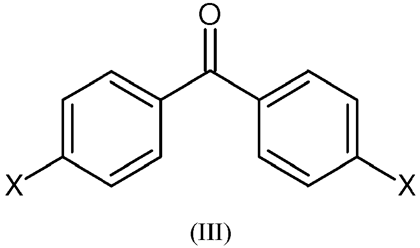 wherein X is as defined above.

Another object of the invention relates to a process for the preparation of a 4,4'-dihalobenzophenone of formula (III): 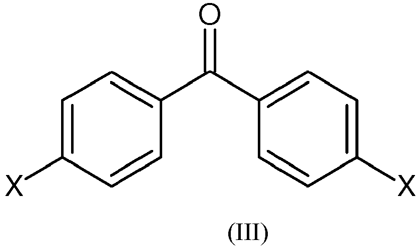 wherein X represents a halogen atom selected from the group consisting of fluorine, chlorine, bromine and iodine,
comprising carrying out the dehydrogenation/aromatization of a compound of formula (I): 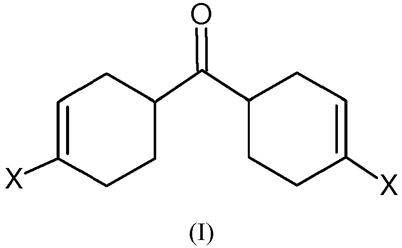 wherein X is as defined above.

According to an embodiment, the dehydrogenation/aromatization is carried out in the presence of an oxidation catalyst, preferably manganese oxide (MnO₂) or copper acetate (Cu(OAc)₂).

According to an embodiment, the dehydrogenation/aromatization is carried out in the presence of a solvent. Examples of suitable solvents are sulfoxides, preferably those chosen among dialkyl sulfoxides wherein each out of the 2 alkyl groups contains from 1 to 6 carbon atoms (especially from 1 to 4 carbon atoms), alkyl phenyl sulfoxides wherein the alkyl group contains from 1 to 6 carbon atoms (especially from 1 to 4 carbon atoms), tetrahydrothiophene 1-oxide and mixtures thereof. Good results were obtained notably when using dimethyl sulfoxide (DMSO) as the solvent.

Another object of the invention relates to a process for the preparation of a 4,4'-dihalobenzophenone of formula (III): 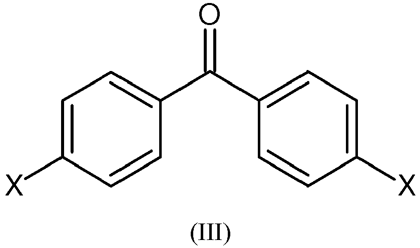 wherein X represents a halogen atom selected from the group consisting of fluorine, chlorine, bromine and iodine,
comprising:
a) providing a compound of formula (I): 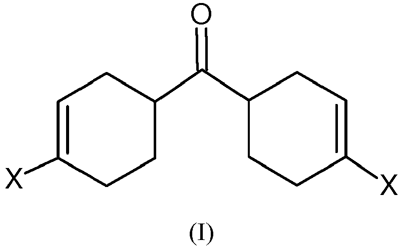 wherein X is as defined above,
b) carrying out the dehydrogenation/aromatization of said compound of formula (I),
c) optionally further isolating compound of formula (III).

According to an embodiment of the invention, compound of formula (I) is prepared by reacting divinylketone with a 2-halobutadiene of formula (II): 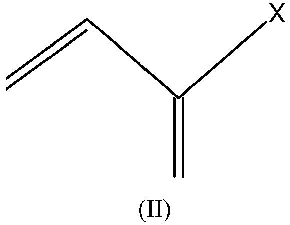 wherein X is as defined above.

As already mentioned, one of the advantages of the invention is to provide a process for the preparation of 4,4'-dihalobenzophenones involving renewable starting materials.

Moreover, it has been surprisingly found that the process for the preparation of compound of formula (I) according to the invention allows satisfactory conversion and selectivity.

### DETAILED DESCRIPTION OF THE INVENTION

The present invention is directed to a compound of formula (I): 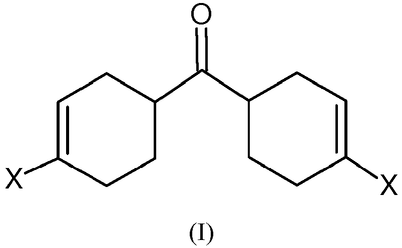 wherein X represents a halogen atom selected from the group consisting of fluorine, chlorine, bromine and iodine.

In a preferred embodiment of the invention, the halogen atom is selected from chlorine and fluorine.

Compound of formula (I) may be prepared from divinylketone and a 2-halobutadiene of formula (II): 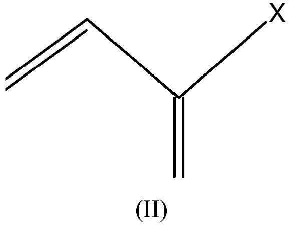 wherein X is as defined above.

Preferably, the 2-halobutadiene of formula (II) is 2-chlorobutadiene (chloroprene) or 2-fluorobutadiene (fluoroprene).

The 2-halobutadiene of formula (II) may be prepared by any conventional chemical reactions known to the one skilled in the art, or may be also commercially available products. In particular, synthesis of fluoroprene is described for example in US2469848, US2437308, US2451612 and US2437148.

Divinylketone can be obtained from formaldehyde and acetone, for example via the Mannich reaction as described in J. Gen. Chem. USSR, Volume 33(5), p. 1512 (1963), from 1,5-dibromopentan-3-one as described in Chem. Eur. J. 2007, 13, pp. 3354-3368, or by oxidation of divinyl carbinol as described in Org. Biomol. Chem., 2010, 8, pp. 751-754. Formula of divinylketone is :

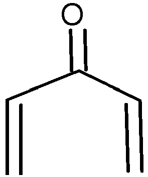

It has been surprisingly found that a Diels-Alder reaction efficiently occurs between the 2-halobutadiene of formula (II) and divinylketone resulting in the formation of a double cycloadduct of formula (I) as defined above. Moreover, it has been surprisingly found that the Diels-Alder reaction between the 2-halobutadiene of formula (II) and divinylketone leads to the formation of compounds of formula (I) with a good conversion, a low amount of by-products and a good selectivity (i.e. para/para).

The present invention therefore also provides a process for the production of a compound of formula (I), comprising reacting divinylketone with a 2-halobutadiene of formula (II).

The Diels-Alder reaction between the 2-halobutadiene of formula (II) and divinylketone leads to the formation of two main structural isomers represented below, one of which being the compound of formula (I):

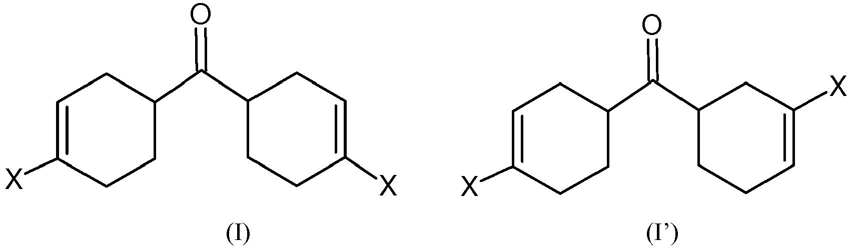

Other isomers might be also obtained in minor amounts, in particular the compound of formula (I"):

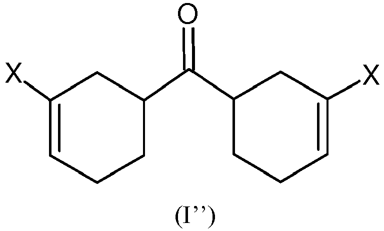

Compounds of formula (I), (I') and (I") can be present as different stereoisomers.

According to an embodiment of the invention, the molar ratio of compounds of formula (I) to compound of formula (I') is of at least 50/50, preferably at least 70/30, more preferably at least 80/20.

According to an embodiment of the invention, the conversion of the Diels-Alder reaction is of at least 50%, preferably at least 70%, more preferably at least 90%.

The Diels-Alder reaction between the 2-halobutadiene of formula (II) and divinylketone can be carried out under usual Diels-Alder conditions known to the person skilled in the art.

The Diels-Alder reaction may be conducted with or without a catalyst. The use of catalysts can improve kinetics and selectivity of the Diels-Alder reaction. Known Diels-Alder catalysts may be used and may include Lewis acids, such aluminium chloride, ethylaluminium dichloride, diethylaluminium chloride, trimethyl aluminium, bismuth (III) chloride, bismuth (III) trifluoromethanesulfonate, boron trifluoride, boron triacetate, cerium (III) chloride, copper (I) trifluoromethanesulfonate, copper (II) chloride, hafnium (IV) chloride, iron (II) chloride, iron (II) acetate, iron (III) chloride, iron (III) acetate, lithium perchlorate, lithium trifluoromethanesulfonate, magnesium bromide, magnesium iodide, magnesium chloride, magnesium perchlorate, scandium (III) trifluoromethanesulfonate, tin (IV) chloride, titanium (IV) chloride, titanium (IV) isopropoxide, N-trimethylsilyl-bis(trifluoromethanesulfonyl)imide, trimethylsilyl trifluoromethanesulfonate, ytterbium (III) trifluoromethanesulfonate, zinc chloride, zinc bromide, zinc iodide, zinc acetate and zirconium (IV) chloride, indium (III) chloride, triphenylborane, Bronsted acids, such as inorganic mineral acids, e.g. sulfuric acid, phosphoric acid, nitric acid, hydrobromic acid or hydrochloric acid, and organic acids such as methane sulfonic acid, p-toluenesulfonic acid or carboxylic acids. One of the preferred Diels-Alder catalysts is iron chloride (FeCl₃).

Alternatively, activated carbon, silica, alumina, silica-alumina, zirconia and zeolites may be used as such or as support for a catalytically active metal or metal compound. Suitable metals or metal compounds include alkali metals, alkaline earth metals, transition metals, noble metals, rare earth metals. The catalysts can be acidic, e.g. by treating supports with phosphoric acid, or by ion exchange of zeolites to render them into their acidic form. Examples of solid catalysts include amorphous silica-alumina, zeolites, preferably zeolites in their H-form, and acidic ion exchange resins. Other suitable catalysts that are liquids or that may be dissolved in the appropriate solvent to yield a homogeneous catalyst environment, include organic and inorganic acids, such as alkane carboxylic acid, arene carboxylic acid, sulfuric acid, phosphoric acid, hydrochloric acid, hydrobromic acid and nitric acid.

The Diels-Alder reaction may be carried out with or without a solvent. Suitable solvents may be selected from pyridine. In case a catalyst is used, it is preferred to use dichloromethane as solvent or to use no solvent at all.

The reaction may be carried out at any suitable temperature, for example from about -80 to about 120 °C, preferably from about -20 to about 100 °C, more preferably from about -10 to about 80 °C, for a time sufficient to convert the starting compounds into the desired Diels-Alder adduct, such as about 10 minutes to about 6 days, preferably about 3 hours to about 4 days, more preferably about 3 hours to about 2 days. The reaction can be carried out at ambient pressure or under increased pressure. In a preferred embodiment, the reaction is carried out at ambient pressure, such as about 1 bar, or at a pressure of up to 10 bars, preferably up to 5 bars, more preferably up to 2 bars.

According to an embodiment of the invention, the molar ratio of the 2-halobutadiene of formula (II) to divinylketone is close to stoichiometry (namely 2/1), or above.

The process according to the invention may comprise a further step of isolation of compound of formula (I). Suitable methods of isolation and purification are known to the person skilled in the art. For example, chromatography techniques, such as liquid chromatography, may be particularly efficient in the process according to the invention.

The present invention also relates to a process for the preparation of a 4,4'-dihalobenzophenone of formula (III): 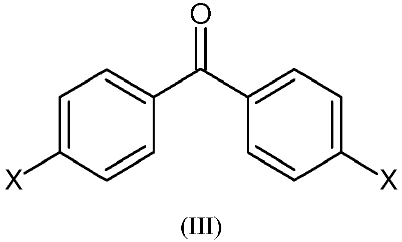 wherein X represents a halogen atom selected from the group consisting of fluorine, chlorine, bromine and iodine, comprising carrying out the dehydrogenation/aromatization of the compound of formula (I) as defined above. Preferably, X is selected from fluorine and chlorine.

The dehydrogenation/aromatization may be represented by the following scheme:

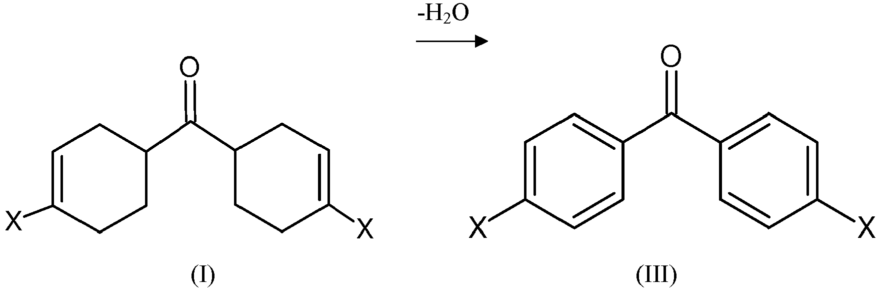

The reaction conditions for aromatization of the compound of formula (I) are known to a person skilled in the art.

Suitable catalysts or reagents to conduct the aromatization reaction may be (i) a copper salt or a copper oxide in presence of a base (such as an alkali metal acetate or alkali metal hydroxide), or (ii) manganese dioxide.

Suitable solvents for the aromatization reaction may be for example chosen from sulfoxides such as DMSO (especially when the catalyst or reagent used to conduct the aromatization reaction is a copper salt or a copper oxide in presence of a base), and from chlorobenzene and toluene (especially when the catalyst or reagent used to conduct the aromatization reaction is manganese dioxide).

The aromatization reaction may be carried out at any suitable temperature, for example from about -10 to about 120 °C, preferably from about -5 to about 80 °C, more preferably from about 0 to about 40 °C.

Another object of the invention relates to a process for producing a 4,4'-dihalobenzophenone of formula (III): 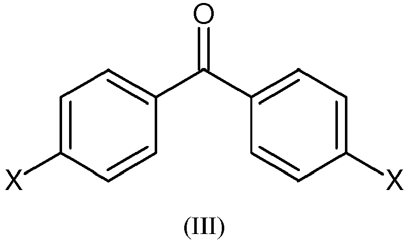 wherein X represents a halogen atom selected from the group consisting of fluorine, chlorine, bromine and iodine, comprising:
a) providing a compound of formula (I) as defined above,
b) carrying out the dehydrogenation/aromatization of said compound of formula (I).

When 4,4'-difluorobenzophenone is desired, it may be prepared by effecting a halogen-fluorine exchanging reaction between compound of formula (III) wherein X is chlorine, bromine or iodine, and an alkali fluoride, such as potassium fluoride, as described in US 4,453,009 and US2002161227. Alternatively, it may also be possible to proceed with said halogen-fluorine exchanging reaction directly on the compound of formula (I) wherein X is chlorine, bromine or iodine, in accordance with the teaching of US4792618.

According to an embodiment of the invention, the compound of formula (I) is obtained by a Diels-Alder reaction between divinylketone and the above-described 2-halobutadiene of formula (II) according to the process previously detailed.

Once the Diels-Alder reaction is completed, isolation of composition of formula (I) may be carried out before step b).

Alternatively, the mixture obtained at the end of the Diels-Alder reaction and comprising structural isomers of formula (I), (I') and (I") can be directly used in the subsequent step of dehydrogenation/aromatization. In this case, a mixture comprising the following structural isomers is obtained after completing the aromatization:

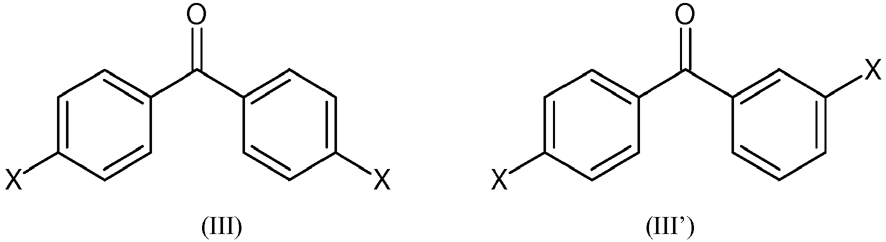

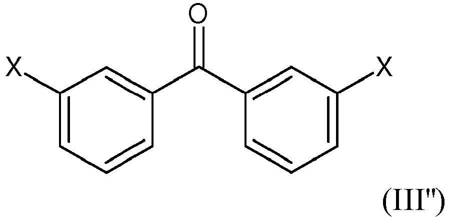

According to an embodiment of the invention, 4,4'-dihalobenzophenone of formula (III) is further isolated by separation methods known to the person skilled in the art. For example, recrystallization may be carried out according to the method described in document US 4,873,372.

Poly(aryl ether ketone)s (PAEK) are a well known class of engineering polymers useful in various fields of scientific and commercial endeavour. PAEK polymers are generally prepared by aromatic nucleophilic substitution of both the halogenides of a 4,4'-dihalobenzophenone (especially, by aromatic nucleophilic substitution of both the fluorides of 4,4'-difluorobenzophenone) by a nucleophilic oxygen atom from a co-monomer. For example, p-hydroquinone, commonly referred to as "hydroquinone" and/or at least one bisphenol (such as bisphenol A, bisphenol S, bisphenol O, 4,4'-dihydroxybiphenyl and mixtures thereof) can be used as the co-monomer; the hydrogen atom from each of the two aromatic hydroxyl groups of p-hydroquinone and/or of the bisphenol is advantageously deprotonated with at least one base (such as NaOH, Na₂CO₃, K₂CO₃ and mixtures thereof) to form a nucleophile which reacts with the 4,4'-dihalobenzophenone (preferably, 4,4'-difluorobenzophenone) to form a PAEK polymer via a nucleophilic substitution mechanism, with the halogen atoms of the 4,4'-dihalobenzophenone acting as leaving groups. Processes for preparing PAEK polymers, including those using a 4,4'-dihalobenzophenone such as 4,4'-difluorobenzophenone, can be found notably in U.S. Pat. Nos. 3,953,400, 3,956,240, 3,928,295, and 4,176,222, all incorporated herein by reference.

Then, facets of the invention relate to :
- the use of a 4,4'-dihalobenzophenone of formula (III) prepared from a compound of formula (I) as previously defined for the manufacture of a poly(aryl ether ketone), in particular poly(ether ether ketone),
- the use of a compound of formula (I) as previously defined for the manufacture of a poly(aryl ether ketone), in particular poly(ether ether ketone), typically with a 4,4'-dihalobenzophenone of formula (III) as synthesis intermediate, and
- the use of a 4,4'-dihalobenzophenone of formula (III) as synthesis intermediate in the manufacture of a poly(aryl ether ketone) [in particular poly(ether ether ketone] from a compound of formula (I) as above defined.

Precisely an object of the present invention concerns a process for the manufacture of a (poly aryl ether ketone), said process comprising the following steps:
i) providing a compound of formula (I) as defined above,
ii) carrying out the dehydrogenation/aromatization of said compound of formula (I) to prepare a 4,4'-dihalobenzophenone of formula (III) as defined above,
iii) optionally, isolating the 4,4'-dihalobenzophenone,
iv) optionally, effecting a halogen-fluorine exchanging reaction between the 4,4'-dihalobenzophenone and an alkali fluoride as explained above ,
v) carrying out the polycondensation of the 4,4'-dihalobenzophenone with at least one aromatic compound comprising 2 hydroxyl groups, in particular with hydroquinone and/or at least one bisphenol, preferably in the presence of at least one base.

Should the disclosure of any patents, patent applications, and publications which are incorporated herein by reference conflict with the description of the present application to the extent that it may render a term unclear, the present description shall take precedence.

The present invention will now be illustrated by the following examples, which are not intended to be limiting.

### EXAMPLES

### a) Diels-Alder reaction of divinylketone with chloroprene

In a carousel tube fitted with a PTFE septum screw cap chloroprene (1.66 g, 18.8 mmol) was weighted. Then dichloromethane (DCM) (2 mL), FeC13 (122 mg, 0.75 mmol) and divinylketone (618 mg, 7.53 mmol) were added. The reaction heat increases the reaction media temperature to the ebullition of DCM. After 1 hour of stirring at room temperature, the reaction media is filtrated through a pad of silica gel washed with DCM. After solvents evaporation, 816 mg of a brown oil (42% yield) is obtained, corresponding to a mixture of double Diels-Alder cycloadducts (compounds of formula (I)). GC and NMR analysis indicated that the product is a 83/17 mixture of 4,4' and 3,4' isomers (very little amount of 3,3' isomers)

MS : 258

¹H and [¹³C] NMR :

The main regioisomer (4,4') is a 50/50 mixture of 2 diastereoisomers (rac/meso) as there is 2 ¹³C pics for each carbon. All ¹H NMR signals are complex multiplets :

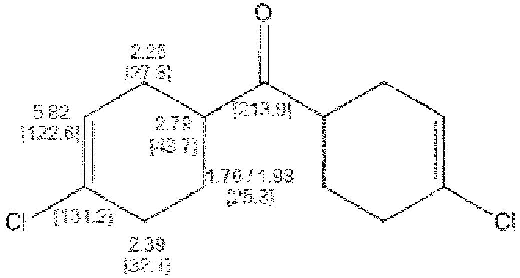

### b) Aromatization of the double Diels-Alder adducts obtained at step a)

In a 50-ml round bottom flask fitted with a reflux condenser was charged 6.1 g of manganese dioxide (activated, Aldrich), 20 mL of chlorobenzene, 1g of potassium carbonate and 450 mg of the cycloadducts obtained from the precedent example. The reaction mixture was stirred at 110°C during 20h. GC analysis of the liquid show the complete conversion into the desired dichlorobenzophenone (compounds of formula (III) in a ratio of 90/10 for the isomers 4,4'/3,4').

## Claims

1. Compound of formula (I): 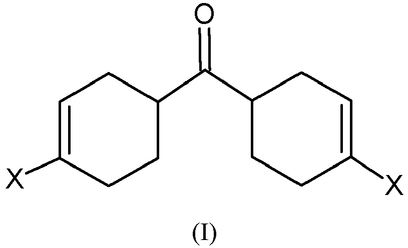 wherein X represents a halogen atom selected from the group consisting of fluorine, chlorine, bromine and iodine.

2. Compound according to claim 1, wherein the halogen atom is selected from fluorine and chlorine.

3. Process for the preparation of a compound of formula (I) as defined in claim 1 or 2, comprising reacting divinylketone with a 2-halobutadiene (II): 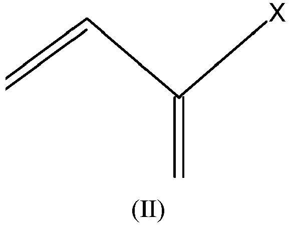 wherein X is as defined above,
and optionally further isolating compound of formula (I).

4. Process according to claim 3, wherein the 2-halobutadiene is 2-chlorobutadiene or 2-fluorobutadiene.

5. Process according to claim 3 or 4, wherein the molar ratio of the 2-halobutadiene of formula (II) to divinylketone is of at least 2/1.

6. Use of a compound of formula (I) as defined in claim 1 or 2, for the preparation of a 4,4'-dihalobenzophenone of formula (III): 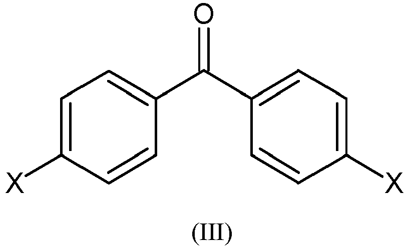 wherein X is as defined above.

7. Process for the preparation of a 4,4'-dihalobenzophenone of formula (III): 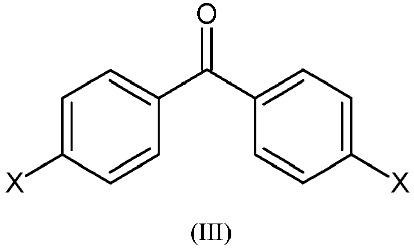 wherein X represents a halogen atom selected from the group consisting of fluorine, chlorine, bromine and iodine,
comprising carrying out the dehydrogenation/aromatization of a compound of formula (I): 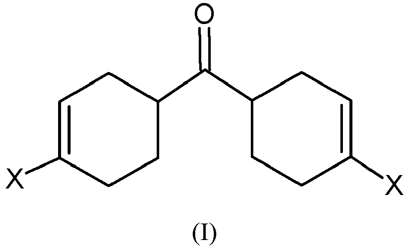 wherein X is as defined above.

8. Process according to claim 7, wherein the dehydrogenation/aromatization is carried out in the presence of a solvent.

9. Process according to claim 7, which comprises:
a) providing compound of formula (I)
b) carrying out the dehydrogenation/aromatization of said compound of formula (I),
c) optionally further isolating 4,4'-dihalobenzophenone of formula (III).

10. Process according to claim 9, wherein compound of formula (I) is prepared by reacting divinylketone with a 2-halobutadiene of formula (II): 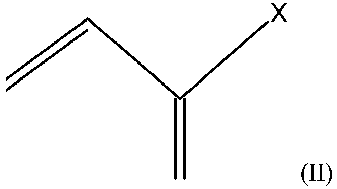 wherein X is as defined above.

11. Use of a compound of formula (I) as defined in claim 1 or 2 for the manufacture of a poly(aryl ether ketone).

12. Use according to claim 11, wherein the poly(aryl ether ketone) is poly(ether ether ketone).

## Patentansprüche

1. Verbindung der Formel (I): 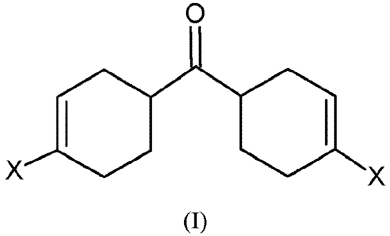 wobei X für ein Halogenatom aus der Gruppe bestehend aus Fluor, Chlor, Brom und Iod steht.

2. Verbindung nach Anspruch 1, wobei das Halogenatom aus Fluor und Chlor ausgewählt ist.

3. Verfahren zur Herstellung einer Verbindung der Formel (I) gemäß Anspruch 1 oder 2, bei dem man Divinylketon mit einem 2-Halogenbutadien (II): 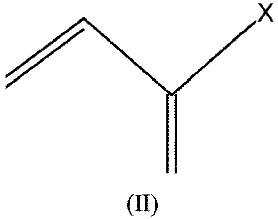 umsetzt, wobei X wie oben definiert ist,
und gegebenenfalls ferner die Verbindung der Formel (I) isoliert.

4. Verfahren nach Anspruch 3, wobei es sich bei dem 2-Halogenbutadien um 2-Chlorbutadien oder 2-Fluorbutadien handelt.

5. Verfahren nach Anspruch 3 oder 4, wobei das Molverhältnis von 2-Halogenbutadien der Formel (II) zu Divinylketon mindestens 2/1 beträgt.

6. Verwendung einer Verbindung der Formel (I) gemäß Anspruch 1 oder 2 zur Herstellung eines 4,4'-Dihalogenbenzophenons der Formel (III): 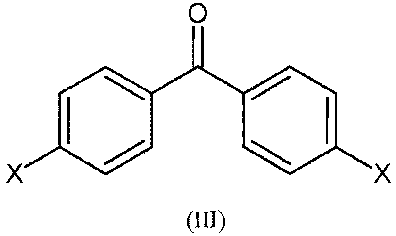 wobei X wie oben definiert ist.

7. Verfahren zur Herstellung eines 4,4'-Dihalogenbenzophenons der Formel (III): 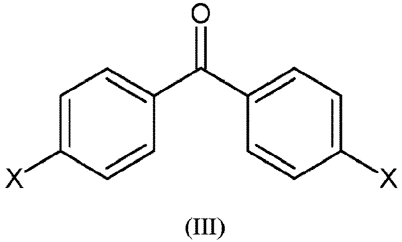 wobei X für ein Halogenatom aus der Gruppe bestehend aus Fluor, Chlor, Brom und Iod steht,
bei dem man die Dehydrierung/Aromatisierung einer Verbindung der Formel (I): 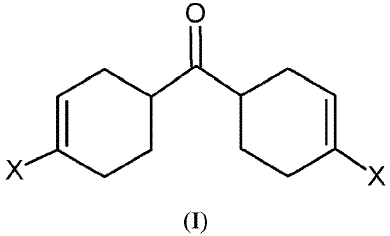 durchführt, wobei X wie oben definiert ist.

8. Verfahren nach Anspruch 7, bei dem man die Dehydrierung/Aromatisierung in Gegenwart eines Lösungsmittels durchführt.

9. Verfahren nach Anspruch 7, bei dem man:
a) die Verbindung der Formel (I) bereitstellt,
b) die Dehydrierung/Aromatisierung der Verbindung der Formel (I) durchführt,
c) gegebenenfalls ferner das 4,4'-Dihalogenbenzophenon der Formel (III) isoliert.

10. Verfahren nach Anspruch 9, bei dem man die Verbindung der Formel (I) durch Umsetzen von Divinylketon mit einem 2-Halogenbutadien der Formel (II) : 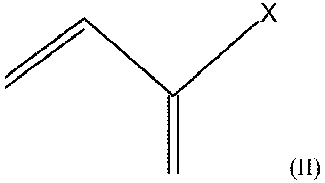 herstellt, wobei X wie oben definiert ist.

11. Verwendung einer Verbindung der Formel (I) gemäß Anspruch 1 oder 2 zur Herstellung eines Poly(aryletherketon)s.

12. Verwendung nach Anspruch 11, wobei es sich bei dem Poly(aryletherketon) um Poly(etheretherketon) handelt.

## Revendications

1. Composé de formule (I) : 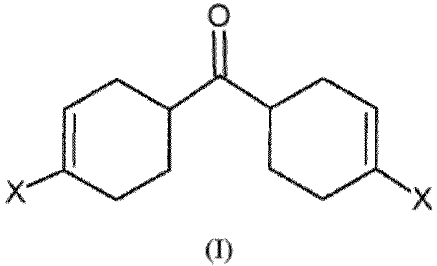 X représentant un atome d'halogène choisi dans le groupe constitué par fluor, chlore, brome et iode.

2. Composé selon la revendication 1, l'atome d'halogène étant choisi parmi fluor et chlore.

3. Procédé pour la préparation d'un composé de formule (I) tel que défini dans la revendication 1 ou 2, comprenant la réaction de divinylcétone avec un 2-halogénobutadiène (II) : 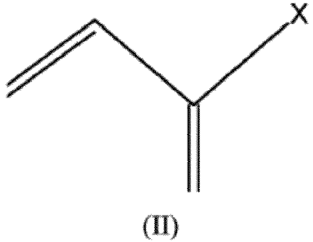 X étant tel que défini ci-dessus,
et éventuellement en outre l'isolement du composé de formule (I).

4. Procédé selon la revendication 3, le 2-halogénobutadiène étant le 2-chlorobutadiène ou le 2-fluorobutadiène.

5. Procédé selon la revendication 3 ou 4, le rapport molaire du 2-halogénobutadiène de formule (II) sur la divinylcétone étant d'au moins 2/1.

6. Utilisation d'un composé de formule (I) tel que défini dans la revendication 1 ou 2, pour la préparation d'une 4,4'-dihalogénobenzophénone de formule (III) : 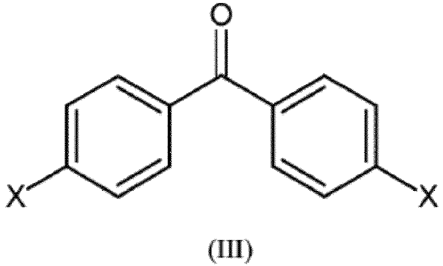 X étant tel que défini ci-dessus.

7. Procédé pour la préparation d'une 4,4'-dihalogénobenzophénone de formule (III) : 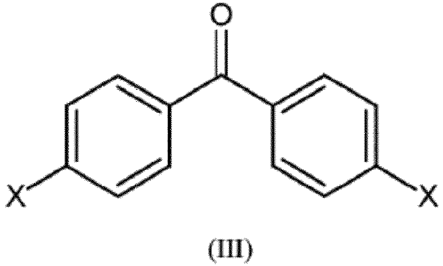 X représentant un atome d'halogène choisi dans le groupe constitué par fluor, chlore, brome et iode, comprenant la réalisation de la déshydrogénation/aromatisation d'un composé de formule (I) : 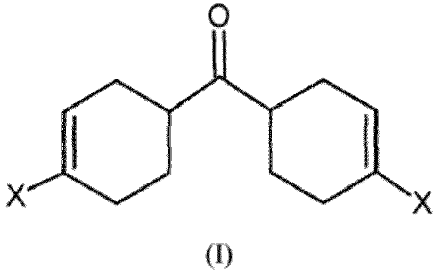 X étant tel que défini ci-dessus.

8. Procédé selon la revendication 7, la déshydrogénation/aromatisation étant réalisée en la présence d'un solvant.

9. Procédé selon la revendication 7, qui comprend :
a) la mise à disposition du composé de formule (I)
b) la réalisation de la déshydrogénation/aromatisation dudit composé de formule (I),
c) éventuellement en outre l'isolement de la 4,4'-dihalogénobenzophénone de formule (III).

10. Procédé selon la revendication 9, le composé de formule (I) étant préparé par mise en réaction de divinylcétone avec un 2-halogénobutadiène de formule (II) : 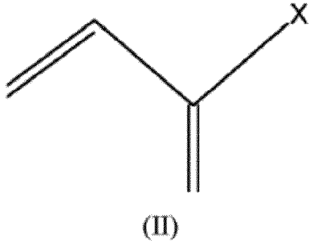 X étant tel que défini ci-dessus.

11. Utilisation d'un composé de formule (I) tel que défini dans la revendication 1 ou 2 pour la fabrication d'une poly(aryléthercétone).

12. Utilisation selon la revendication 11, la poly(aryléthercétone) étant une poly(étheréthercétone).
